# FASCICULE DE BREVET EUROPEEN

(11) **EP 2 475 338 B1**
(45) Date de publication et mention de la délivrance du brevet: **13.07.2016**
(21) Numéro de dépôt: 10754686.3
(22) Date de dépôt: 06.09.2010
(51) Int. Cl.: A61F 5/01, B25J 9/00, A61H 1/02

(54) **MECANISME D'EPAULE POUR ORTHESE**
SCHULTERMECHANISMUS FÜR ORTHESE
SHOULDER MECHANISM FOR ORTHESIS

(30) Priorité: 09.09.2009 FR 0904313
(43) Date de publication de la demande: 18.07.2012
(73) Titulaire: Commissariat à l'Énergie Atomique et aux Énergies Alternatives, 75015 Paris (FR)
(72) Inventeur: GARREC, Philippe, 91190 Gif-sur-Yvette (FR)
(74) Mandataire: Parzy, Benjamin Alain
(86) Numéro de dépôt international: PCT/EP2010/005452
(87) Numéro de publication internationale: WO 2011/029564

(56) Documents cités:
- WO-A1-2008/131563
- WO-A2-95/32842
- WO-A2-2008/031023
- DE-A1- 19 940 603
- FR-A1- 2 917 323
- US-A- 4 669 451
- US-A1- 2007 225 620
- US-A1- 2008 009 771
- MICHAEL SCOTT LISZKA: 'MECHANICAL DESIGN OF A ROBOTIC ARM EXOSKELETON FOR SHOULDER REHABILITATION (MASTERS THESIS)', [en ligne] 01 Janvier 2006, UNIVERSITY OF MARYLAND, page 7PP, XP055140657 Extrait de l'Internet: <URL:http://drum.lib.umd.edu/bitstream/1903 /4236/1/umi-umd-4064.pdf> [extrait le 2014-09-16]

## Description

L'invention est relative à un mécanisme d'épaule pour orthèse.

### ARRIERE-PLAN TECHNOLOGIQUE DE L'INVENTION

On connaît des mécanismes d'épaule comportant un premier élément solidaire de l'utilisateur du mécanisme. Ce premier élément peut être fixe, ou porté par l'utilisateur. Un deuxième élément est articulé sur le premier élément selon un premier axe d'articulation sensiblement horizontal, s'étendant selon une direction longitudinale s'étendant d'avant en arrière par référence à l'utilisateur. Un troisième élément est articulé sur le deuxième élément selon un deuxième axe d'articulation sensiblement vertical lorsque le mécanisme est au repos (c'est-à-dire quand le bras de l'utilisateur s'étend le long de son corps), le deuxième axe d'articulation étant perpendiculaire au premier axe d'articulation. Enfin, le bras proprement dit est articulé au troisième élément selon un troisième axe d'articulation de nouveau sensiblement horizontal, et sensiblement perpendiculaire aux premier et deuxième axes d'articulation.

De tels mécanismes d'épaule sont connus, par exemple, du document WO 2008/131563 A1 ou encore du document *"*Mechanical Design of a Robotic Arm Exoskeleton for Shoulder Rehabilitation" (M. S. Liszka, Master of Science, 2006), qui divulguent tous deux un mécanisme selon le préambule de la revendication 1 annexée.

Il est connu des mécanismes d'épaule dont les trois articulations sont réalisées au moyen de pivots successifs. Cependant, de tels mécanismes présentent en général des interférences avec le bras de l'utilisateur lors de mouvement du bras. En particulier, le deuxième pivot, celui qui réalise l'articulation du troisième élément sur le deuxième élément, est porté par le deuxième élément pour s'étendre par-dessus l'épaule, de sorte que lors d'une abduction du bras, le deuxième pivot vient très rapidement au voisinage de la tête de l'utilisateur limitant ainsi grandement l'amplitude de l'abduction permise.

Il a été proposé de réaliser l'articulation du troisième élément sur le deuxième élément au moyen d'une glissière s'étendant selon un arc de cercle, qui s'étend horizontalement au repos pour faire le tour de l'épaule de l'utilisateur. Une telle disposition présente de nombreux avantages, notamment celui de minimiser les interférences entre le mécanisme et l'utilisateur lors de mouvements courants du bras.

Cependant, une telle disposition peut présenter certains inconvénients. L'abduction du bras de l'utilisateur à des angles proches de 90 degrés amène la glissière au voisinage du visage de l'utilisateur, ce qui peut se révéler gênant. Par ailleurs, pour des raisons pratiques de réalisation, le rayon de la glissière peut difficilement descendre sous les 100 millimètres, ce qui empêche la réalisation de mécanismes d'orthèses adaptées aux enfants. En outre, la glissière subit des flexions importantes, ce qui peut induire une fragilité mécanique, ainsi qu'une augmentation des frottements.

### OBJET DE L'INVENTION

L'invention a pour objet de proposer un mécanisme d'orthèse évitant l'usage d'une glissière en arc de cercle, tout permettant des débattements importants.

### BREVE DESCRIPTION DE L'INVENTION

En vue de la réalisation de ce but, on propose un mécanisme d'épaule pour orthèse comme défini dans la revendication 1. Ce mécanisme comporte successivement :
- un premier élément fixe par rapport à l'utilisateur ;
- un deuxième élément articulé sur le premier élément selon un premier axe d'articulation
- un troisième élément articulé sur le deuxième élément selon un deuxième axe d'articulation
- un bras qui est articulé au troisième élément selon un troisième axe d'articulation. Les

Les axes d'articulation s'étendent selon des directions définissant un repère non-orthogonal. Une telle disposition minimise les risques d'interférence avec l'utilisateur, et permet de nouveau l'utilisation de paliers pour réaliser les articulations. Cette disposition permet en outre un plus grand débattement des éléments articulés, offrant ainsi un plus grand volume de travail. Il conviendra, comme dans les autres mécanismes, d'éviter toute singularité par alignement de deux axes d'articulation du mécanisme.

Selon l'invention, les axes d'articulation sont inclinés par rapport aux directions verticales et horizontales lorsque le bras de l'utilisateur est au repos, comme défini dans la revendication 1.

### PRESENTATION DES FIGURES

L'invention sera mieux comprise à la lumière de la description qui suit des figures des dessins annexés, parmi lesquelles :

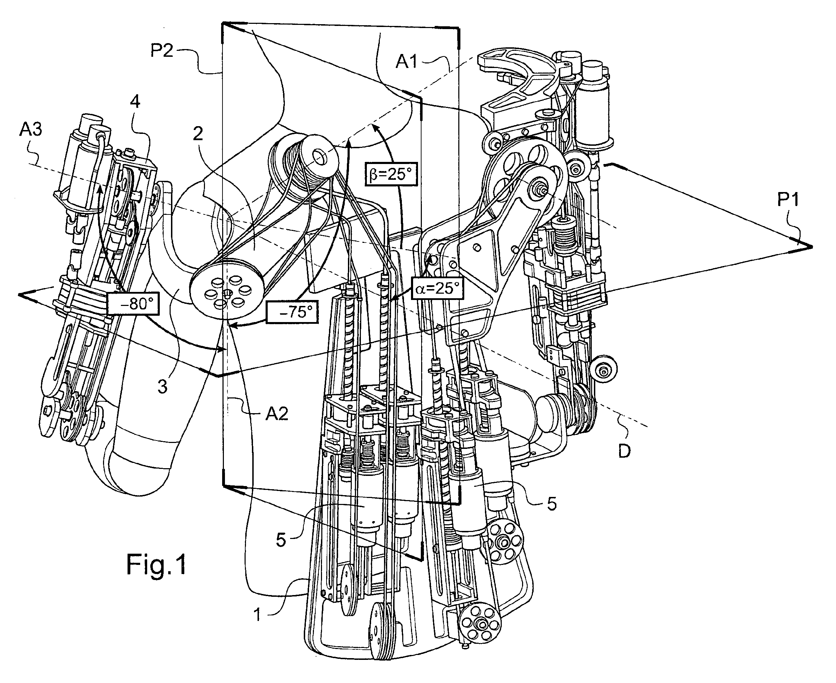

- la figure 1 est une vue en perspective de derrière d'un utilisateur portant une orthèse pourvue d'un mécanisme d'épaule selon un mode particulier de réalisation de l'invention ;
- la figure 2 est une vue de perspective, de derrière, mais plus proche de la vue de côté, du même utilisateur ;
- la figure 3 est une vue de dessus du même utilisateur

### DESCRIPTION DETAILLEE DES FIGURES

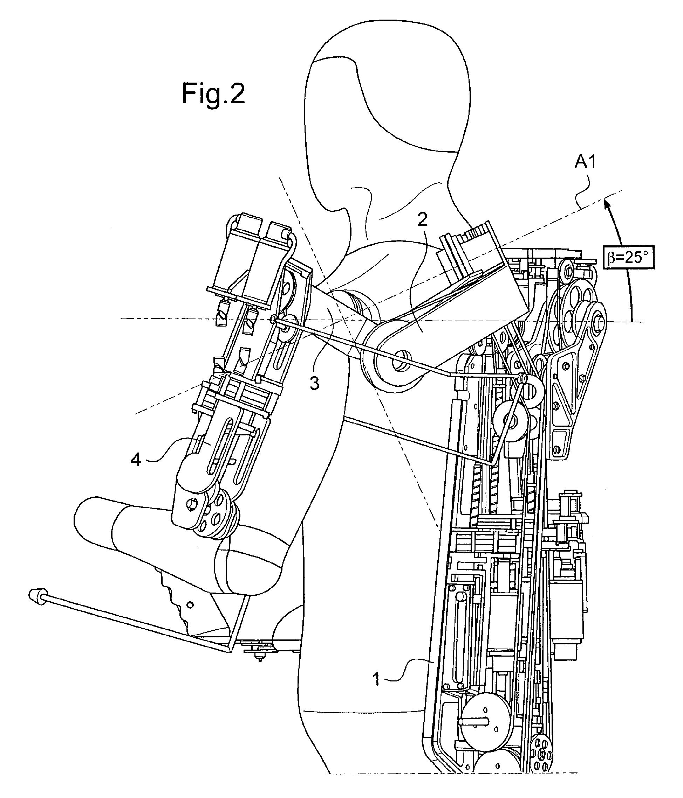

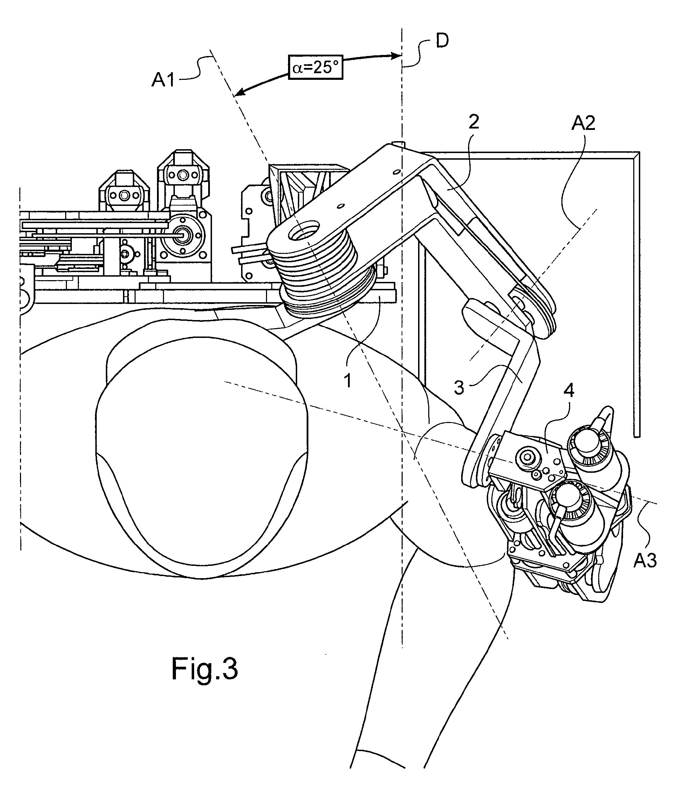

Le mécanisme d'épaule illustré sur les figures comporte tout d'abord un premier élément 1, ou dossier, porté ici directement par l'utilisateur, et s'étendant contre le dos de celui-ci. Sur ce premier élément 1 est articulé un deuxième élément 2 selon un premier axe d'articulation A1. Selon l'invention, l'axe d'articulation A1 est incliné, de la façon suivante. Si P1 est un plan horizontal passant par le centre de l'épaule, et D une direction longitudinale passant dans le plan P et s'étendant d'avant en arrière par référence à l'utilisateur, alors l'axe d'articulation A1 fait un angle α de 25 degrés dans le plan P1 (angle visible en vraie grandeur sur la figure 3), et un angle β de 25 degrés dans un plan vertical P2 (angle visible en vraie grandeur sur la figure 2), par rapport à une horizontale.

Puis un troisième élément 3 est articulé sur le deuxième élément selon un deuxième axe d'articulation A2 qui est orienté selon un angle γ de 75 degrés par rapport au premier axe d'articulation A1.

Enfin, le bras 4 de l'orthèse est articulé sur le troisième élément selon un troisième axe d'articulation A3 qui s'étend selon un angle 80 degrés par rapport au deuxième axe d'articulation A2. Ainsi et selon une caractéristique essentielle de l'invention, les trois axes d'articulation s'étendent selon des directions définissant un repère non-orthogonal.

Les trois articulations sont ici matérialisées par des liaisons pivots réalisées à l'aide de paliers de pivotement.

Les trois axes d'articulation coïncident en un point qui est le centre présumé de l'épaule de l'utilisateur. Cependant, cette disposition n'est pas requise dans la cadre de l'invention, et les trois axes d'articulation pourront simplement converger au voisinage de ce centre présumé sans être strictement concourants.

Ces dispositions permettent un débattement étendu du bras, sans utilisation de glissières circulaires. L'actionnement des degrés de liberté de pivotement autour des premier et deuxième axes d'articulation est de préférence réalisé à l'aide de vérins à câbles fixés 5 sur le dossier 1, et dont les câbles sont reçus ou renvoyés en tant que de besoin par des poulies concentriques aux diverses articulations.

L'invention n'est bien sûr pas limitée à ce qui vient d'être décrit. En particulier, les valeurs d'angles sont données à titre indicatif et seront à adapter à la morphologie de l'utilisateur. Une fourchette de plus ou moins 10 degrés autour des valeurs indiquées permettra, de prendre en compte la plupart des morphologies d'épaule.

## Revendications

1. Mécanisme d'épaule pour orthèse comportant successivement :
- un premier élément (1) fixe par rapport à l'utilisateur ;
- un deuxième élément (2) articulé sur le premier élément selon un premier axe d'articulation (A1) ;
- un troisième élément (3) articulé sur le deuxième élément selon un deuxième axe d'articulation (A2) ;
- un bras (4) qui est articulé au troisième élément selon un troisième axe d'articulation (A3) ;
les axes d'articulations (A1, A2, A3) s'étendant selon des directions définissant un repère non orthogonal, le dispositif étant **caractérisé en ce que**, lorsque le bras de l'utilisateur est au repos,
le premier axe d'articulation (A1) s'étend selon un angle (α) dans une fourchette de plus ou moins 10 degrés autour d'un angle de 25 degrés dans un plan horizontal (P1) passant par un centre présumé de l'épaule et par rapport à une direction (D) longitudinale passant dans le plan horizontal (P1) et s'étendant d'avant en arrière par référence à l'utilisateur, le premier axe d'articulation (A1) s'étendant également selon un angle (β) dans une fourchette de plus ou moins 10 degrés autour d'un angle de 25 degrés dans un plan vertical (P2), par rapport au plan horizontal (P1).

2. Mécanisme d'épaule pour orthèse selon la revendication 1, dans lequel le deuxième axe d'articulation (A2) s'étend selon un angle (γ) dans une fourchette de plus ou moins 10 degrés autour d'un angle de 75 degrés par rapport au premier axe d'articulation (A1).

3. Mécanisme d'épaule pour orthèse selon la revendication 2, dans lequel le troisième axe d'articulation (A3) s'étend selon un angle dans une fourchette de plus ou moins 10 degrés autour d'un angle de 80 degrés par rapport au deuxième axe d'articulation (A2).

4. Mécanisme d'épaule pour orthèse selon l'une des revendications précédentes, dans lequel les articulations sont matérialisées par des paliers de pivotement.

## Patentansprüche

1. Schultermechanismus für Orthese, nacheinander umfassend:
- ein erstes Element (1), das in Bezug auf einen Nutzer ortsfest ist;
- ein zweites Element (2), das an dem ersten Element um eine erste Gelenkverbindungssachse (A1) angelenkt ist;
- ein drittes Element (3), das an dem zweiten Element um eine zweite Gelenkverbindungsachse (A2) angelenkt ist;
- einen Arm (4), der an dem dritten Element um eine dritte Gelenkverbindungsachse (A3) angelenkt ist;
wobei sich die Gelenkverbindungsachsen (A1, A2, A3) in Richtungen erstrecken, die ein nicht-orthogonales Bezugssystem definieren, wobei die Vorrichtung **dadurch gekennzeichnet ist, dass**, wenn der Arm des Nutzers im Ruhezustand ist,
sich die erste Gelenkverbindungsachse (A1) in einem Winkel (α) in einer Spanne von plus/minus 10 Grad um einen Winkel von 25 Grad in einer horizontalen Ebene (P1) erstreckt, die durch einen angenommenen Mittelpunkt der Schulter verläuft, sowie in Bezug auf eine Längsrichtung (D), die in der horizontalen Ebene (P1) verläuft und sich in Bezug auf den Nutzer von vorne nach hinten erstreckt, wobei sich die erste Gelenkverbindungsachse (A1) ebenfalls in einem Winkel (β) in einer Spanne von plus/minus 10 Grad um einen Winkel von 25 Grad in einer in Bezug auf die horizontale Ebene (P1) vertikalen Ebene (P2) erstreckt.

2. Schultermechanismus für Orthese nach Anspruch 1, wobei sich die zweite Gelenkverbindungsachse (A2) in einem Winkel (γ) in einer Spanne von plus/minus 10 Grad um einen Winkel von 75 Grad in Bezug auf die erste Gelenkverbindungsachse (A1) erstreckt.

3. Schultermechanismus für Orthese nach Anspruch 2, wobei sich die dritte Gelenkverbindungsachse (A3) in einem Winkel in einer Spanne von plus/minus 10 Grad um einen Winkel von 80 Grad in Bezug auf die zweite Gelenkverbindungsachse (A2) erstreckt.

4. Schultermechanismus für Orthese nach einem der vorhergehenden Ansprüche, wobei die Gelenkverbindungen durch Schwenklager umgesetzt sind.

## Claims

1. A shoulder mechanism for an orthosis comprising in succession:
· a first element (1) that is stationary relative to the user;
· a second element (2) that is hinged to the first element about a first hinge axis (A1);
· a third element (3) that is hinged to the second element about a second hinge axis (A2); and
· an arm (4) that is hinged to the third element about a third hinge axis (A3);
the hinge axes (A1, A2, A3) extending in directions that define a frame of reference that is non-orthogonal the shoulder mechanism being **characterized in that**, when the user's arm is at rest,
the first hinge axis (A1) extends at an angle (α) in a range of plus or minus 10 degrees about an angle of 25 degrees relative to a horizontal plane (P1) containing a presumed center of the shoulder and relative to a longitudinal direction (D) contained in the horizontale plane (P10) and extending from front to back relative to the user, , the first hinge axis (A1) also extending at an angle (β) in a range of plus or minus 10 degrees about an angle of 25 degrees in a vertical plane (P2) relative to the horizontal plane (P1).

2. A shoulder mechanism for an orthosis according to claim 1, wherein the second hinge axis (A2) extends at an angle (γ) in a range of plus or minus 10 degrees about an angle of 75 degrees relative to the first hinge axis (A1).

3. A shoulder mechanism for an orthosis according to claim 2, wherein the third hinge axis (A3) extends at an angle in a range of plus or minus 10 degrees about an angle of 80 degrees relative to the second hinge axis (A2).

4. A shoulder mechanism for an orthosis according to any of the preceding claims, wherein the hinges are embodied by pivot bearings.
